(19)

Europäisches Patentamt

European Patent Office

Office européen des brevets

(11)  **EP 4 163 638 A2**

(12)  **EUROPEAN PATENT APPLICATION**

(43) Date of publication:
   **12.04.2023   Bulletin 2023/15**

(21) Application number: **22200313.9**

(22) Date of filing: **07.10.2022**

(51) International Patent Classification (IPC):
   **G01N 33/68** *(2006.01)*

(52) Cooperative Patent Classification (CPC):
   **G01N 33/6863;** G01N 2333/5406; G01N 2333/715;
   G01N 2333/7158; G01N 2800/202; G01N 2800/52;
   G01N 2800/54

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **KH MA MD TN**

(30) Priority:   **07.10.2021   EP 21201480**

(71) Applicant: **Beiersdorf Aktiengesellschaft
   20245 Hamburg (DE)**

(72) Inventors:
   • **AL, Benjamin
     20354 Hamburg (DE)**
   • **SEIDEL, Judith
     13507 Berlin (DE)**
   • **HOLZSCHECK, Nicholas
     22549 Hamburg (DE)**
   • **REUTER, Jörn Hendrik
     24558 Henstedt-Ulzburg (DE)**

(74) Representative: **Haseltine Lake Kempner LLP
   Bürkleinstrasse 10
   80538 München (DE)**

(54)  **METHOD FOR PREDICTING ATOPIC DERMATITIS RELAPSE**

(57)   The present invention relates to a method for predicting atopic dermatitis relapse, a method for determining a suitable dermatological agent or cosmetic for preventing or delaying atopic dermatitis relapse, and the use of one or more biological protein marker(s) for predicting atopic dermatitis.

EP 4 163 638 A2

**Description**

FIELD OF THE INVENTION

The present invention discloses a method for predicting atopic dermatitis relapse, a method for determining a suitable dermatological agent or cosmetic agent for preventing or delaying atopic dermatitis relapse, and the use of one or more biological protein marker(s) for predicting atopic dermatitis.

BACKGROUND OF THE INVENTION

Atopic dermatitis (AD) or eczema is a widespread, relapsing skin disease that is characterized by skin lesions at specific body sites, resulting in symptoms such as redness and itching. Atopic dermatitis is particularly common in children but can occur at any age.

Although there is no known cure for atopic dermatitis, there are treatments and self-care measures available to alleviate the symptoms of atopic dermatitis. Due to the rather complex nature of atopic dermatitis, however, there is no single therapeutic treatment that is suitable to treat all (or a majority) of patients suffering from atopic dermatitis. Even with the availability of a large variety of treatment options to control the symptoms of atopic dermatitis, lesions recur in most patients within weeks after cessation of treatment. However, relapse patterns differ from patient to patient.

To improve atopic dermatitis treatment, there is a need to move towards precision medicines due to the complexity of the disease. The active lesional state has been well-researched in the last few decades as can be seen, for example, in Renert-Yuval, Y., et al., Biomarkers in atopic dermatitis-a review on behalf of the International Eczema Council. J Allergy Clin Immunol, 2021. 147(4): p. 1174-1190.e1. For example, a systemic $T_H2$ skewed immune component has been found to drive the disease, resulting in blood biomarkers that characterize the active disease state.

Despite this research, factors that trigger new relapses are poorly understood and the known studies focus on patients in the active lesional state. This means that using currently known methods, a specific treatment can only be identified once the patient is suffering from the symptoms of atopic dermatitis.

There is a need to better identify the onset of atopic dermatitis to allow identification of a suitable treatment at an early stage of the relapse. This would allow therapeutic strategies to be adapted to the individual patient accordingly and intervention could occur before any clinical symptoms appear.

It is therefore desirable to provide a method for predicting atopic dermatitis relapse. In particular, it is desirable to provide a method that allows for predicting atopic dermatitis relapse in patients at an early stage of new outbreaks.

SUMMARY OF THE INVENTION

The present invention is defined in the appended claims.

In accordance with a first aspect, there is provided a method for predicting atopic dermatitis relapse in a patient in remission, wherein the method comprises:

a) determining the level of one or more biological marker(s) in a skin sample of the said patient; and

b) predicting atopic dermatitis relapse by comparing the level of the one or more biological marker(s) obtained from step a) with empirically determined data.

In accordance with a second aspect, there is provided a method for determining a suitable dermatological agent or cosmetic agent for preventing or delaying atopic dermatitis relapse, comprising the method of the first aspect followed by selecting an emollient to apply to the skin. An emollient is generally known in the art as a component of cosmetic compositions. In some cases, emollients have specific characteristics, such as lipids and waxes, which interact with lipids of the skin due to their lipophilic character. An emollient may be a liquid or cream which can be applied to the skin to improve the skin's properties, such as softness, and/or to reduce pain.

In accordance with a third aspect, there is provided a use of one or more biological marker(s) for predicting atopic dermatitis relapse in a patient in remission or exhibiting mild symptoms of atopic dermatitis according to the method the first aspect.

Certain embodiments of the present invention may provide one or more of the following advantages:

• desired ability to predict atopic dermatitis relapse before the active lesion state;
• desired reduction of level of suffering (including psychosocial pressure) due to delay or prevention of relapse;
• desired reduction of use of therapeutic agents associated with discomfort or side effects (such as corticosteroids or calcineurin inhibitors);
• desired ease of sample collection from the individual;

- desired prediction of specific site;
- desired speed of obtaining data level of biological protein markers;
- desired accuracy of prediction.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

It is understood that the following description and references to the figures concern exemplary embodiments of the present invention and shall not be limiting the scope of the claims.

DETAILED DESCRIPTION

The present invention is based on the surprising finding that the level of certain biological markers in skin and/or blood samples can be used to predict atopic dermatitis relapse. For example, gene expression levels in skin samples can be used to predict atopic dermatitis relapse. In a further example an increase in certain biological protein markers in blood can be used to predict atopic dermatitis relapse. This prediction of atopic dermatitis relapse occurs even before the patient has exhibited any symptoms of atopic dermatitis.

The present methods of prediction are targeted towards patients that have previously been diagnosed with and/or treated for atopic dermatitis. In particular, the patients according to the present method are in remission from atopic dermatitis or exhibit mild symptoms of atopic dermatitis. Patients in "remission" may also be referred to as "asymptomatic patients", i.e. patients that are not suffering any symptoms of atopic dermatitis at the time the method is carried out. As used herein, patients "exhibiting mild symptoms" of atopic dermatitis are participants with an EASI (Eczema Area and Severity Index) score of below 5.

As used herein, a "biological marker" is a measurable indicator of atopic dermatitis. The "biological marker" may be a "biological protein marker" or a "biological mRNA marker". The term "biological protein marker" may be used interchangeably with "protein marker" or "protein biomarker". The term "biological mRNA marker" may be used interchangeably with "mRNA marker" or "mRNA biomarker".

In one method disclosed herein, prediction of atopic dermatitis relapse is determined using skin samples. This method predicts atopic dermatitis relapse at a specific local site correlating to where the skin sample is harvested.

In certain embodiments, the mRNA marker levels in the skin samples are measured, which can be determined, for example, by using PCRs (polymerase chain reactions), microarrays or any form of sequencing such as a single cell RNA sequencing (scRNA-seq). In one example scRNA-seq is used to determine the mRNA marker levels, using the method described below.

In certain embodiments the protein maker levels in the skin are measured. The protein marker levels can be determined using ELISA, Multiplex immunoassays or lateral flow tests.

In certain embodiments, the skin sample may be obtained from the patient using any known skin harvesting method. Harvesting a sample from the individual may be carried out using full thickness biopsies, suction blistering, punch biopsy, shave biopsy, skin swabs, tape stripping or during any surgical procedure such as plastic surgery, lifting, grafting, or the like. In certain embodiments non-invasive harvesting methods are used, such as skin swabs and tape stripping.

The skin samples may be taken from the epidermis or dermis.

In certain embodiments the skin samples may comprise biological markers, such as biological mRNA markers. In certain embodiments, the one or more biological mRNA marker(s) of the present method are independently selected from the mRNA markers in Table 1.

Table 1: List of biological mRNA markers

| No. | mRNA | No. | mRNA | No. | mRNA | No. | mRNA |
|-----|-------|-----|--------|-----|--------|-----|--------|
| 1 | SPRR2A | 21 | SPRR2G | 41 | EMP1 | 61 | PHLDA2 |
| 2 | S100A8 | 22 | FOSB | 42 | DSC2 | 62 | MIDN |
| 3 | S100A9 | 23 | S100P | 43 | CDKN1A | 63 | HSPH1 |
| 4 | LCE3D | 24 | SPRR2E | 44 | IFITM3 | 64 | MCL1 |
| 5 | DEFB4A | 25 | HEPHL1 | 45 | ZFP36L1 | 65 | CD44 |
| 6 | SPRR2D | 26 | MT-ND4 | 46 | TMSB10 | 66 | NDRG1 |

(continued)

| No. | mRNA | No. | mRNA | No. | mRNA | No. | mRNA |
|-----|---------|-----|---------|-----|---------|-----|--------|
| 7 | SPRR2B | 27 | S100A10 | 47 | LYPD3 | 67 | SAT1 |
| 8 | ACTB | 28 | ANXA1 | 48 | FOXQ1 | 68 | GJB2 |
| 9 | LCE3E | 29 | MT-CO2 | 49 | ODC1 | 69 | CALML3 |
| 10 | KRT16 | 30 | PLAUR | 50 | LIMA1 | 70 | KRT15 |
| 11 | KRT6A | 31 | HBEGF | 51 | ITGA2 | 71 | CREG1 |
| 12 | S100A7 | 32 | CNFN | 52 | ERRFI1 | 72 | KLK11 |
| 13 | SERPINB4 | 33 | MT-CYB | 53 | TRIM29 | 73 | MT1E |
| 14 | TM4SF1 | 34 | SLURP2 | 54 | YBX3 | 74 | CALML5 |
| 15 | MT2A | 35 | SOD2 | 55 | PRDM1 | 75 | LOR |
| 16 | SERPINB2 | 36 | MT-ND2 | 56 | VKORC1 | 76 | RBM39 |
| 17 | AREG | 37 | SLC38A2 | 57 | AQP3 | 77 | DAPL1 |
| 18 | EGR1 | 38 | MT-ATP6 | 58 | AHNAK | 78 | LGALS7 |
| 19 | SPRR5 | 39 | S100A2 | 59 | KRT17 | 79 | KRT2 |
| 20 | MT-ND3 | 40 | CLDN4 | 60 | CCL27 | | |

The present inventors have surprisingly found that the biological markers of the skin, such as the biological mRNA markers listed in Table 1, may be suitable for predicting atopic dermatitis relapses. The markers of Table 1 were identified by analysing empirically determined data from ex-lesional skin biopsies. The identification of suitable markers involves screening healthy control skin samples and comparing them to relapsing skin samples. Biological mRNA markers were selected that showed a notable difference between the healthy control skin sample and the relapsing skin sample, as disclosed below. These markers were identified as suitable for predicting atopic dermatitis relapses, such as the biological mRNA markers of Table 1.

As disclosed herein, a "healthy control skin sample" refers to a skin sample taken from a site of a subject, which has recently been affected by lesions but is now in remission and does *not* show symptoms of atopic dermatitis within the observed time frame. A "relapsing skin sample" refers to a skin sample taken from a site of a subject, which has recently been affected by lesions but is now in remission and develops symptoms of atopic dermatitis within the observed time frame, such as is itching and redness. The observed time frame is preferably within two months after taking the sample. The healthy control skin sample and the relapsing skin sample can in some examples be from a single subject, or from multiple subjects, also referred to as a "cohort".

Once biological markers suitable for predicting atopic dermatitis relapse are identified, the baseline value of this marker can be established. The baseline values of the relapsing cohort ($value_{relapse}$) and the healthy or non-relapsing cohort ($value_{non-relapse}$) as disclosed herein is the gene expression of a particular marker in the relapse skin sample, and the healthy control skin sample, respectively. These values can be measured as the transcript of the gene of interest per million gene transcripts in total within a cell (short: transcript per million = TPM)). The gene expression ratio of the baseline ($ratio_{base}$) can then be determined according to equation (1):

$$(1) \quad ratio_{base} = \frac{value_{relapse}}{value_{non-relapse}}$$

In equation (1), the gene expression of the relapsing cohort ($value_{relapse}$) is compared to the gene expression of the healthy or non-relapsing cohort ($value_{non-relapse}$). As disclosed herein, $value_{non-relapse}$ may be used interchangeably with $value_{base}$ and $value_{healthy}$. The relapsing cohort comprises all the patients relapsing within the observed time frame. The non-relapsing cohort comprises all the patients *not* relapsing within the observed time frame.

One example to show how the empirical data is determined is presented in the following. First of all, skin samples are collected from a range of individuals that were recently affected by atopic dermatitis lesions, but who are currently in remission. The skin was analysed, using scRNA-seq (10X Genomics) to quantify the gene expression of each cell present in the sample. The data was analyzed as indicated in the method disclosed below. From this analysis gene expression values of patients that experienced a relapse on the observed site in the next two months were obtained and compared with individuals that did not experience a relapse in the same time frame to generate a gene expression ratio of the baseline (ratio_base).

The baseline values for the non-relapsing cohort (value_non-relapse) and gene expression ratios of the baseline (ratio_base) of the biological mRNA markers of Table 1 are presented in Table 2.

Table 2: Baseline values for the non-relapsing cohort (value_non-relapse) and gene expression ratios of the baseline (ratio_base) of biological mRNA markers

| No. | mRNA | value_non-relapse | ratio_base | No. | mRNA | Value_non-relapse | ratio_base |
|---|---|---|---|---|---|---|---|
| 1 | SPRR2A | 0.10 | 20.28 | 41 | EMP1 | 4.39 | 1.59 |
| 2 | S100A8 | 4.72 | 12.54 | 42 | DSC2 | 1.83 | 1.57 |
| 3 | S100A9 | 2.41 | 5.94 | 43 | CDKN1A | 2.89 | 1.56 |
| 4 | LCE3D | 0.49 | 5.79 | 44 | IFITM3 | 0.67 | 1.56 |
| 5 | DEFB4A | 0.00 | 5.41 | 45 | ZFP36L1 | 6.90 | 1.54 |
| 6 | SPRR2D | 0.50 | 5.11 | 46 | TMSB10 | 4.93 | 1.54 |
| 7 | SPRR2B | 0.21 | 4.97 | 47 | LYPD3 | 8.50 | 1.52 |
| 8 | ACTB | 18.96 | 3.97 | 48 | FOXQ1 | 0.77 | 1.52 |
| 9 | LCE3E | 0.12 | 3.58 | 49 | ODC1 | 0.84 | 1.51 |
| 10 | KRT16 | 5.33 | 3.41 | 50 | LIMA1 | 2.97 | 1.50 |
| 11 | KRT6A | 3.83 | 3.36 | 51 | ITGA2 | 0.89 | 1.48 |
| 12 | S100A7 | 5.38 | 2.84 | 52 | ERRFI1 | 2.69 | 1.48 |
| 13 | SERPINB4 | 0.33 | 2.54 | 53 | TRIM29 | 3.41 | 1.47 |
| 14 | TM4SF1 | 2.44 | 2.46 | 54 | YBX3 | 7.04 | 1.47 |
| 15 | MT2A | 5.81 | 2.27 | 55 | PRDM1 | 0.53 | 1.47 |
| 16 | SERPINB2 | 9.09 | 2.06 | 56 | VKORC1 | 0.39 | 1.46 |
| 17 | AREG | 2.98 | 1.91 | 57 | AQP3 | 14.88 | 1.46 |
| 18 | EGR1 | 2.63 | 1.89 | 58 | AHNAK | 4.33 | 1.46 |
| 19 | SPRR5 | 1.15 | 1.88 | 59 | KRT17 | 9.72 | 1.46 |
| 20 | MT-ND3 | 16.09 | 1.87 | 60 | CCL27 | 3.31 | 1.45 |
| 21 | SPRR2G | 8.90 | 1.86 | 61 | PHLDA2 | 4.87 | 1.44 |
| 22 | FOSB | 3.14 | 1.85 | 62 | MIDN | 1.51 | 1.44 |
| 23 | S100P | 0.69 | 1.79 | 63 | HSPH1 | 2.90 | 1.43 |
| 24 | SPRR2E | 5.80 | 1.78 | 64 | MCL1 | 3.72 | 1.43 |
| 25 | HEPHL1 | 0.04 | 1.75 | 65 | CD44 | 6.00 | 1.43 |
| 26 | MT-ND4 | 10.94 | 1.75 | 66 | NDRG1 | 2.49 | 1.43 |
| 27 | S100A10 | 6.48 | 1.71 | 67 | SAT1 | 5.01 | 1.42 |
| 28 | ANXA1 | 9.01 | 1.71 | 68 | GJB2 | 1.25 | 1.42 |
| 29 | MT-CO2 | 32.55 | 1.71 | 69 | CALML3 | 1.68 | 0.69 |
| 30 | PLAUR | 0.42 | 1.70 | 70 | KRT15 | 5.54 | 0.69 |
| 31 | HBEGF | 1.39 | 1.68 | 71 | CREG1 | 0.84 | 0.69 |
| 32 | CNFN | 1.39 | 1.68 | 72 | KLK11 | 1.17 | 0.69 |
| 33 | MT-CYB | 20.03 | 1.67 | 73 | MT1E | 2.37 | 0.67 |

(continued)

| No. | mRNA | value_non-relapse | ratio_base | No. | mRNA | Value_non-relapse | ratio_base |
|-----|------|-------------------|------------|-----|------|-------------------|------------|
| 34 | SLURP2 | 1.07 | 1.66 | 74 | CALML5 | 8.75 | 0.66 |
| 35 | SOD2 | 2.31 | 1.65 | 75 | LOR | 36.25 | 0.65 |
| 36 | MT-ND2 | 7.16 | 1.61 | 76 | RBM39 | 3.32 | 0.62 |
| 37 | SLC38A2 | 7.30 | 1.61 | 77 | DAPL1 | 2.49 | 0.60 |
| 38 | MT-ATP6 | 16.32 | 1.60 | 78 | LGALS7 | 6.66 | 0.48 |
| 39 | S100A2 | 30.15 | 1.60 | 79 | KRT2 | 12.20 | 0.35 |
| 40 | CLDN4 | 1.94 | 1.60 | | | | |

The data presented in Table 2 can be used as the empirically determined data according to aspect 1 of the present invention. Predicting atopic dermatitis relapse in a patient can then be carried out by measuring the gene expression value for an individual ($value_x$) followed by calculating the gene expression ratio of the individual ($ratio_x$) according to equation (2):

$$(2) \quad ratio_x = \frac{value_x}{value_{non-relapse}}$$

The gene expression ratio of the individual ($ratio_x$) is then compared with the gene expression ratio of the baseline ($ratio_{base}$). For some of the biological markers, gene expression is *increased* in relapsing cases compared to non-relapsing cases (i.e. upregulated; $ratio_{base} > 1$). For some of the biological markers, gene expression is *decreased* in relapsing cases compared to non-relapsing cases (i.e. downregulated; $ratio_{base} < 1$). Following equations (3) to (6), it can then be predicted whether an individual will relapse or not, thereby taking into consideration whether the gene in question is upregulated or downregulated as explained above.

$$(3) \quad \text{if } ratio_{base} > 1 \text{ and } ratio_x > ratio_{base} \rightarrow relapse$$

$$(4) \quad \text{if } ratio_{base} > 1 \text{ and } ratio_x < ratio_{base} \rightarrow no\ relapse$$

$$(5) \quad \text{if } ratio_{base} < 1 \text{ and } ratio_x < ratio_{base} \rightarrow relapse$$

$$(6) \quad \text{if } ratio_{base} < 1 \text{ and } ratio_x > ratio_{base} \rightarrow no\ relapse$$

The method described above using skin samples can also be applied to establish the time to relapse. For example, it may be determined in a relapse will occur in, e.g., less than 7 days, or less than 10 days.

In a further method, the prediction of atopic dermatitis relapse as disclosed herein is determined using blood samples, wherein the level of one or more biological protein marker(s) are compared with empirically determined data representing a correlation between the level of said one or more biological marker(s) and atopic dermatitis relapse. The biological protein marker levels in the blood can be determined, for example, by a Multiplex assay. In one example the Multiplex assay used is the Bio-Plex Multiplex Immunoassay System, Bio-Rad®. In some examples, the Multiplex assay is carried out according to the protocol in the manufacture's "Quick Guide" - Bio-Plex Pro™ Human Cytokine Screening Panel, Quick Guide" instruction manual #10000092045.

In certain embodiments, the blood may be obtained from a patient by several methods, including venipuncturing sampling and arterial sampling.

In the present invention the blood samples may comprise biological protein markers. In certain embodiments, the one or more biological protein marker(s) of the present method are independently selected from the protein markers in Table 3

Table 3: List of biological protein markers

| No. | Protein | No. | Protein | No. | Protein | No. | Protein |
|---|---|---|---|---|---|---|---|
| 1 | CCL21 | 11 | CXCL1 | 21 | CXCL11 | 31 | CCL23 |
| 2 | CXCL13 | 12 | CXCL2 | 22 | CCL2 | 32 | CXCL16 |
| 3 | CCL27 | 13 | CCL1 | 23 | CCL8 | 33 | CXCL12 |
| 4 | CXCL5 | 14 | IFN$\gamma$ | 24 | CCL13 | 34 | CCL17 |
| 5 | CCL11 | 15 | IL1$\beta$ | 25 | CCL22 | 35 | CCL25 |
| 6 | CCL24 | 16 | IL4 | 26 | MIF | 36 | TNF |
| 7 | CCL26 | 17 | IL8 | 27 | CXCL9 | 37 | IL9 |
| 8 | CX3CL1 | 18 | IL10 | 28 | CCL3 | 38 | IL18 |
| 9 | CXCL6 | 19 | IL16 | 29 | CCL15 | 39 | M-CSF |
| 10 | GM-CSF | 20 | CXCL10 | 30 | CCL19 | 40 | CCL5 |

In certain embodiments, one or more biological protein marker(s) is/are selected from IL-4, CCL27, CCL5 and CCL8. For example, the level of one of the biological protein marker IL-4, CCL27, CCL5 or CCL8 is determined to carry out the method of prediction according to the present invention. In certain embodiments, the levels of two or more of the biological protein markers selected from IL-4, CCL27, CCL5 and CCL8 are determined to carry out the method of prediction according to the present invention. For example, two, three or four of the biological protein markers selected from IL-4, CCL27, CCL5 and CCL8 are determined to carry out the method of prediction according to the present invention.

IL-4 is the $T_H2$ cytokine which is very prominently associated with *active* atopic dermatitis and atopic dermatitis severity. CCL27 is a chemoattractant for memory T cells to the skin, which are important producers of the $T_H2$ cytokines IL-4 and IL-13. CCL5 has been reported in one study to be upregulated in spontaneously healed atopic dermatitis skin as opposed to active lesional and healthy skin. CCL8 has not previously been associated with atopic dermatitis. For all of these markers IL-4, CCL27, CCL5 or CCL8, not one of them has been associated with atopic dermatitis relapse. It would, therefore, not have been expected that one or more of these markers would be suitable for predicting atopic dermatitis relapses.

Once the level of the biological protein markers has been determined by the method disclosed herein, the levels are compared with empirically determined data. The empirically determined data represents a correlation between the said one or more biological proteins marker(s) and atopic dermatitis relapse.

The empirical data is determined by collecting blood samples from a range of individuals that had recently been affected by lesions. The serum was analysed, using the Bio-Plex Multiplex Immunoassay System, Bio-Rad®, to quantifying the serum proteins of Table 1. The data for IL-4 was analyzed with Student's t test and CCL27, CCL5 and CCL8 were analyzed with Mann-Whitney U tests since only IL-4 was normally distributed. From this analysis a "cut off point" was determined for each biological protein marker. This "cut off point" distinguishes between whether a relapse occurred within a week of the test, or more than a week from the test. Using this method, the cut off points can be applied to predict whether a relapse is to happen within a week if the level for IL-4 is >= 51 pg/ml; the level for CCL27 >= 1028 pg/ml; the level for CCL5 <= 9558 pg/ml; and/or the level for CCL8 <= 69 pg/ml.

In certain embodiments, the comparison of detected levels of one or more biological protein marker(s) with the empirically determined data indicates a time to relapse. In certain embodiments the time to relapse is 7 days or less. In certain embodiments the time to relapse is more than 7 days.

In the method according to the second aspect, namely a method for determining a suitable dermatological agent or cosmetic agent for preventing or delaying atopic dermatitis relapse, the suitable dermatological agent or cosmetic agent may be any known agent that is known to alleviate the symptoms of atopic dermatitis. In some examples, the agent is an emollient which is applied to the skin.

In certain embodiments, the method may have one or more of the following effects:

- ability to predict atopic dermatitis relapse in a patient in remission from atopic dermatitis;
- ability to predict atopic dermatitis relapse in a patient exhibiting mild symptoms of atopic dermatitis;
- good accuracy of prediction of atopic dermatitis relapse;
- good reliability of correlation between level of biological protein marker(s) and empirical data;
- low cost of predicting atopic dermatitis relapse;
- ability to avoid symptoms of atopic dermatitis by applying emollient to skin based on prediction.

The present disclosure may be described by one or more of the following paragraphs:

1. A method for predicting atopic dermatitis relapse in a patient in remission, or exhibiting mild symptoms of atopic dermatitis, wherein the method comprises:

a) determining the level of one or more biological protein marker(s) in a sample of blood of the said patient;
b) predicting atopic dermatitis relapse by comparing the level of the one or more biological protein marker(s) with empirically determined data representing a correlation between the level of said one or more biological protein marker(s) and atopic dermatitis relapse.

2. The method according to paragraph 1, wherein the one or more biological protein marker(s) is/are selected from Table 1.
3. The method according to paragraph 1 or paragraph 2, wherein the one or more biological protein marker(s) is/are selected from IL-4, CCL27, CCL5 and CCL8.
4. The method according to any preceding paragraph, wherein the comparison of detected levels of one or more biological protein marker(s) with the empirically determined data indicates a time to relapse.
5. The method according to paragraph 4, wherein the time to relapse is 7 days or less.
6. The method according to paragraph 4, wherein the time to relapse is more than 7 days.
7. The method according to any one of paragraphs 1 to 6, wherein the sample of blood comprises whole blood, plasma and/or serum.
8. A method for determining a suitable dermatological agent or cosmetic agent or preventing or delaying atopic dermatitis relapse, comprising the method of any one of paragraphs 1 to 7 followed by selecting an emollient to apply to the skin.
9. Use of one or more biological protein marker(s) for predicting atopic dermatitis relapse in a patient in remission, or exhibiting mild symptoms, of atopic dermatitis according to the method of any one of paragraphs 1 to 7.
10. The use according to paragraph 9, wherein the one or more biological protein marker(s) is/are selected from the list in Table 1.
11. The use according to paragraph 9 or paragraph 10, wherein the one or more biological marker(s) is/are selected from IL-4, CCL27, CCL5 and CCL8.

The foregoing description is directed to particular embodiments of the present invention for the purpose of illustrating it. It will be apparent, however, to one skilled in the art that many modifications and variations to the embodiments described herein are possible and the scope of the present invention is as defined in the appended claims.

EXAMPLES

To illustrate and test the methods disclosed herein, the following data is provided.

**Example 1** - **Skin samples**

Empirical data was generated from skin sample biopsies of 22 patients. Prior to the samples being taken, the patients were topically treated until the lesions at the skin site of interest were healed, and one to two additional weeks passed for the treatment effects to abate. Skin biopsies with a diameter of about 4 mm were then taken at the former lesional site (now in remission) and the skin biopsies were prepared for scRNA-seq. The samples were processed and analysed according to the protocol described in detail below. After taking the biopsy, the patients documented if they developed relapses or not at the biopsied site in a daily diary for the next two months.

Out of 22 patients, 13 patients experienced relapses at the biopsied site while 9 out of 22 did not. For the analysis we compared the relapsing with the non-relapsing cohort and calculated the expression ratio for all detectable genes. Genes fulfilling predefined criteria of difference and quality (for details see below) were assessed as differentiating genes which would be able to distinguish if a patient would relapse or not within the next weeks.

Droplet based scRNA-sequencing

The skin samples were analysed using droplet-based single-cell RNA sequencing (scRNA-seq; from 10X Genomics). This method captures the expression of genes in each single cell of a sample. First, the collected skin biopsies were freshly processed with the Whole Skin Dissociation Kit (Miltenyi-Biotec) and prepared for loading on the Chromium Controller (10X Genomics; Single Cell 3' Library & Gel Bead Kit v3.1) according to the manufacturers' instructions. Quality-controlled mRNA expression libraries were pooled and sequenced on an Illumina NovaSeq6000 sequencer using S4 (300 cycles) flowcell kits (#20028312; Illumina). The 10x Genomics CellRanger pipeline (v3.0.2) was used with default parameters, except for the setting of expected cells (--expect-cells) which was set to 10,000. Alignment was conducted by use of the Human reference dataset 'refdata-gex-GRCh38-2020-A' provided by 10X Genomics.

Analysis of scRNA-seq data

Downstream analysis of the scRNA-seq data was performed in R (v. 4.0.3). To reduce ambient background contamination from lysed cells, the data was first cleaned with a regular processing workflow using SoupX (v. 1.5.2) with an estimated contamination fraction set to 0.2. Next, doublets containing RNA from more than one cell were identified and filtered using scDblFinder (v. 1.4.2) run with default parameters. Further processing of the data was performed using Seurat (v. 4.0.2): Cells were filtered based on calculated quality criteria, excluding cells with less than 200 or more than 6000 expressed genes as well as cells with more than 15 % mitochondrial reads. Next, the data was normalized (Log-Normalization using a scale factor of 10,000) and the 3000 most variable features calculated using an implementation of the variance-stabilizing transformation (vst) provided by Seurat. Data from all samples was then integrated following the calculation of suitable integration anchors calculated using the first 20 dimensions from a canonical correlation analysis, otherwise the default parameters were used. Following this, the 5000 most variable features were calculated based on the integrated data slot (using the vst method), and the data was normalized and scaled in preparation for dimensionality reduction using principal component analysis (PCA). A suitable number of principal components (PCs) for downstream analyses was determined using visual inspection of elbow plots and set to 15. Next, clustering of cells was performed using a nearest neighbour search using the above mentioned 15 PCs, followed by cluster analysis using the original Louvain community detection algorithm with an initial resolution of 0.2. Annotation of cell types was performed based on manual inspection of the resulting clusters and predominantly expressed cells. For visualization purposes, a non-linear dimensionality reduction was calculated from the integrated data using the Uniform Manifold Approximation and Projection algorithm (UMAP) implemented in Seurat, based on the top 15 PCs and using 20 nearest neighbours for local approximations in the manifold structure.

Selection of predictive marker genes

Genes that are differentially expressed between relapsing and non-relapsing individuals were calculated from all cells using generalized linear models using the algorithm variant implemented in the R package MAST (v. 1.16.0), comparing the TPM values of the relapsing cohort ($value_{relapse}$) with the non-relapsing cohort ($value_{non-relapse}$) and resulting in the calculated ratios ($ratio_{base}$) described above. Genes with expression in less than 10 % of cells and with an absolute log2 fold-change of less than 0.5 were excluded from the analysis. During modelling, gene expression was adjusted for age and sex of donors, as well as sequencing depth of individual cells and their mitochondrial and ribosomal RNA content. Genes with a p-value of less than 0.05 after multiple testing adjustment and an absolute log2 fold-change of at least 0.5 were considered differentially regulated and predictive for an imminent relapse. Predictive genes were then visually inspected regarding their expression in healthy and lesional AD skin, both on single-cell and sample-level, followed by further refining the set of markers.

**Example 2 - Blood samples**

Blood was taken from two patients, patient X and patient Y, who had recently experienced the symptoms of atopic dermatitis, such as an active flare, but were in remission at the timepoint of sampling. The serum from the sampled blood was analysed using the Bio-Plex Multiplex Immunoassay System, Bio-Rad®. The data for IL-4, CCL27, CCL5 and CCL8 obtained was compared against the empirical data. As described above, the empirical data provides the following cut-off points to predict whether a relapse is to happen within a week of sampling. A relapse is predicted to occur within a week if IL-4 is >= 51 pg/ml; the level for CCL27 >= 1028 pg/ml; the level for CCL5 <= 9558 pg/ml; and/or the level for CCL8 <= 69 pg/ml.

The data for patient X and patient Y is provided in Table 4:

Table 4: Examples for atopic dermatitis relapse prediction

| | Biomarker level (in pg/ml) | | | | Predicted time of relapse | Actual time of relapse |
|---|---|---|---|---|---|---|
| | IL4 | CCL27 | CCL8 | CCL5 | | |
| patient X | 58.2 | 1209.4 | 81.6 | 8901.2 | <7 days | Day 1 |
| patient Y | 31.9 | 662.1 | 119.5 | 15142.1 | >7 days | Day 8 |

According to all marker levels patient X was predicted to relapse in week 1, and patient Y in week 2 or later. Indeed, patient X relapsed at day 1 and patient Y at day 8. This data shows that there is the present method provide a good correlation between the predicted time of relapse and the actual time of relapse. Similar results were observed in other patients.

**Claims**

1. A method for predicting atopic dermatitis relapse in a patient in remission, wherein the method comprises:

   a) determining the level of one or more biological marker(s) in a skin sample of the said patient; and
   b) predicting atopic dermatitis relapse by comparing the level of the one or more biological marker(s) obtained from step a) with empirically determined data.

2. The method according to claim 1, wherein the skin sample is obtained from a specific skin site.

3. The method according to claim 1 or claim 2, wherein the skin site has previously been treated for atopic dermatitis.

4. The method according to any one of the preceding claims, wherein the skin sample is harvested *in vivo.*

5. The method according to claim 4, where the skin sample is harvested using a method selected from full thickness biopsies, suction blistering, punch biopsy, shave biopsy, skin swabs, tape stripping or during any surgical procedure such as plastic surgery, lifting, grafting, or the like.

6. The method according to any one of the preceding claims, wherein the one or more biological marker(s) are selected from biological protein markers and/or biological mRNA markers.

7. The method according to any one of the preceding claims, wherein the one or more biological marker(s) is/are biological mRNA markers.

8. The method according to claim 7, wherein the one or more biological mRNA marker(s) is/are selected from Table 1.

9. The method according to any preceding claim, wherein the empirically determined data is based on the corresponding baseline value for the non-relapsing cohort ($value_{non-relapse}$) and the corresponding gene expression ratio of the baseline ($ratio_{base}$) of the one or more biological marker(s).

10. The method according to claim 9, wherein the gene expression ratio of the baseline ($ratio_{base}$) is calculated according to equation (1):

$$(1)\ ratio_{base} = \frac{value_{relapse}}{value_{non-relapse}}$$

**11.** A method for determining a suitable dermatological agent or cosmetic agent or preventing or delaying atopic dermatitis relapse, comprising the method of any one of claims 1 to 10 followed by selecting an emollient to apply to the skin.

**12.** Use of one or more biological marker(s) for predicting atopic dermatitis relapse in a patient in remission according to the method of any one of claims 1 to 10.

**13.** The use according to claim 12, wherein the one or more biological marker(s) is/are biological mRNA markers.

**14.** The use according to claim 13, wherein the one or more biological mRNA protein marker(s) is/are selected from the list in Table 1.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RENERT-YUVAL, Y. et al.** Biomarkers in atopic dermatitis-a review on behalf of the International Eczema Council. *J Allergy Clin Immunol,* 2021, vol. 147 (4), 1174-1190.e1